# EUROPEAN PATENT APPLICATION

(11) **EP 4 489 015 A1**
(43) Date of publication of application: **08.01.2025**
(21) Application number: 23382697.3
(22) Date of filing: 07.07.2023
(51) Int. Cl.: G16H 20/17, G16H 40/63, A61B 5/00, A61B 5/145

(54) **METHODS AND SYSTEMS FOR MEAL EVENT DETECTION AND MEDICATION BOLUS CALCULATION**

(71) Applicant: Roche Diabetes Care GmbH, 68305 Mannheim (DE); F. Hoffmann-La Roche AG, 4070 Basel (CH); International Business Machines Corporation, Armonk, New York 10504 (US)
(72) Inventor: Galley, Paul Joseph, Indianapolis, 46250 Indiana (US); Ringemann, Christian, 68305 Mannheim (DE); Leppaaho, Eemili, Helsinki (FI); Klopfenstein, Yannick, 8048 Zurich (CH); Lustenberger, Patrick, 8048 Zurich (CH)
(74) Representative: Altmann Stößel Dick Patentanwälte PartG mbB

(57) **Abstract**

A method for medication bolus calculation has been developed. The method includes receiving physiological measurements from an analyte sensor coupled to a user, receiving data corresponding to at least one meal event including a timestamp corresponding to when a meal was consumed, identifying a missed meal event time corresponding to a meal consumed by the user but not to the timestamp of any meal event, the recommended bolus being calculated based, at least in part, upon an length of time elapsed from the missed meal event time to a current time, and generating an output message indicating the recommended bolus of the medication.

## Description

### TECHNICAL FIELD

This disclosure is directed to systems and methods for the identification of meal events and generation of warnings and medication bolus recommendations for meals that are not properly recorded in a diabetes management system and to identification of hypoglycemic episodes and recovery meals generally and, more particularly, to systems and methods for the identification of meal events and calculation of insulin bolus dosages for Persons with Diabetes.

### BACKGROUND

Some Persons with Diabetes (PwDs) require daily injections of insulin to maintain healthy glycemic levels, which are typically measured as glucose levels. In PwDs whose glucose levels are considered to be "in range", certain events such as the consumption of a meal produce an increase in glucose that requires the dosing of an insulin bolus to prevent hyperglycemia. Conversely, a PwD may experience a hypoglycemic episode in which the glucose level drops below the recommended range, and the PwD consumes a meal or administers a hormone such as glucagon to return the glucose level to be in range. PwDs who need to inject medications often use a diabetes management software application to provide bolus calculations to determine the proper dosage of insulin other medication that should be administered with a meal, or to track hypoglycemic episodes. The diabetes management software provides benefits not only for short term management of diabetes, but also for long term health of the PwD because the diabetes management application can store a record of the glucose levels and instances and patterns of hyperglycemia and hypoglycemia over longer periods of time that health care professionals may analyze to provide improved treatment for the PwD.

While existing diabetes management software can provide many benefits to PwDs, in some situations the PwDs do not use the diabetes management software consistently. For example, in some situations the PwD consumes a meal and forgets to use the diabetes management software to record the meal and to administer a proper bolus of insulin or other medication with the meal. In other situations, the PwD experiences hypoglycemia and needs to consume a meal or administer a medication such as glucagon to return glucose to an in range level. Because hypoglycemic episodes often cause disorientation and may occur at night, the PwD may forget to use the diabetes management application to record the recovery from the hypoglycemic episode. Consequently, improvements to systems and methods that improve the identification of meal events, hypoglycemia events, and the calculation of medication boluses for PwDs would be beneficial.

### SUMMARY

In one embodiment, a method for medication bolus calculation has been developed. The method includes receiving, with a processor, a plurality of physiological measurements from an analyte sensor, the plurality of physiological measurements being received as a time series of physiological measurements from the analyte sensor coupled to a user, identifying, with the processor, a missed meal event corresponding to a meal consumed by the user, calculating, with the processor, a recommended bolus to administer in response to the missed meal event, the recommended bolus being calculated based, at least in part, upon an length of time elapsed from the timestamp of the missed meal event to a current time, and generating, with an output device coupled to the processor, an output message indicating the recommended bolus of the medication. The identifying of the missed meal event further includes generating a timestamp for the missed meal event based on a change in the plurality of physiological measurements corresponding to a time of the meal, identifying an elevated level of the physiological measurements above a predetermined threshold occurring subsequent to the timestamp, and identifying that the timestamp for the missed meal event does not correspond to a timestamp of any of one or more additional meal events stored in a memory operatively coupled to the processor.

In another embodiment, a method for unrecorded meal identification in a diabetes management system has been developed. The method includes receiving, with a processor, a plurality of physiological measurements from an analyte sensor, the plurality of physiological measurements being received as a time series of physiological measurements from the analyte sensor coupled to a user, identifying, with the processor, an unrecorded meal event corresponding to a meal consumed by the user, generating, with an output device coupled to the processor, an output to alert the user to the unrecorded meal event, and storing, with the processor, a record of the unrecorded meal event in a memory in response to receiving a confirmation via an input device coupled to the processor. The identifying of the unrecorded meal event further includes generating a timestamp for the unrecorded meal event based on a change in the plurality of physiological measurements corresponding to a time of the meal, identifying a reduction in the plurality of physiological measurements occurring subsequent to the timestamp corresponding to a physiological reaction to administration of a medication bolus, and identifying that the timestamp for the unrecorded meal event does not correspond to a timestamp of any of one or more additional meal events stored in a memory operatively coupled to the processor.

In another embodiment, a method for recovery meal event detection in a diabetes management system has been developed. The method includes receiving, with a processor, a plurality of physiological measurements from an analyte sensor, the plurality of physiological measurements being received as a time series of physiological measurements from the analyte sensor coupled to a user, identifying, with the processor, a recovery meal event corresponding to a meal consumed by the user, generating, with an output device coupled to the processor, an output to alert the user to the recovery meal event, and storing, with the processor, a record of the recovery meal event in a memory in response to receiving a confirmation via an input device coupled to the processor. The identifying of the recovery meal event further includes generating a timestamp for the recovery meal event based on a change in the plurality of physiological measurements corresponding to a time of the meal, identifying at least a portion of the plurality of physiological measurements occurring prior to the timestamp falling beneath a predetermined threshold, and identifying that the timestamp for the recovery meal event does not correspond to a timestamp of any of one or more additional meal events stored in a memory operatively coupled to the processor.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 is a schematic diagram of an illustrative embodiment of a diabetes management system that includes a glucose sensor, electronic device, and an optional medication delivery device.
FIG. 2 is a block diagram of a process for missed meal event detection and bolus recommendation that is performed using the system of FIG. 1.
FIG. 3 is a block diagram of a process for recovery meal event detection that is performed using the system of FIG. 1.
FIG. 4 is a block diagram of a process for detecting and recording recovery meal events.
FIG. 5 is a graph depicting a series of glucose measurements that are generated during an unrecorded meal event using the system of FIG. 1.
FIG. 6 is a graph depicting a series of glucose measurements that are generated during a missed meal event using the system of FIG. 1.
FIG. 7 is a graph depicting a series of glucose measurements that are generated during a recovery meal event using the system of FIG. 1.

### Detailed Description

These and other advantages, effects, features and objects are better understood from the following description. In the description, reference is made to the accompanying drawings, which form a part hereof and in which there is shown by way of illustration, not limitation, embodiments of the inventive concept. Corresponding reference numbers indicate corresponding parts throughout the several views of the drawings.

While the inventive concept is susceptible to various modifications and alternative forms, exemplary embodiments thereof are shown by way of example in the drawings and are herein described in detail. It should be understood, however, that the description of exemplary embodiments that follows is not intended to limit the inventive concept to the particular forms disclosed, but on the contrary, the intention is to cover all advantages, effects, and features falling within the spirit and scope thereof as defined by the embodiments described herein and the embodiments below. Reference should therefore be made to the embodiments described herein and embodiments below for interpreting the scope of the inventive concept. As such, it should be noted that the embodiments described herein may have advantages, effects, and features useful in solving other problems.

The devices, systems and methods now will be described more fully hereinafter with reference to the accompanying drawings, in which some, but not all embodiments of the inventive concept are shown. Indeed, the devices, systems and methods may be embodied in many different forms and should not be construed as limited to the embodiments set forth herein; rather, these embodiments are provided so that this disclosure will satisfy applicable legal requirements.

Likewise, many modifications and other embodiments of the devices, systems and methods described herein will come to mind to one of skill in the art to which the disclosure pertains having the benefit of the teachings presented in the foregoing descriptions and the associated drawings. Therefore, it is to be understood that the devices, systems and methods are not to be limited to the specific embodiments disclosed and that modifications and other embodiments are intended to be included within the scope of the embodiments. Although specific terms are employed herein, they are used in a generic and descriptive sense only and not for purposes of limitation.

Unless defined otherwise, all technical and scientific terms used herein have the same meaning as commonly understood by one of skill in the art to which the disclosure pertains. Although any methods and materials similar to or equivalent to those described herein can be used in the practice or testing of the methods, the preferred methods and materials are described herein.

Moreover, reference to an element by the indefinite article "a" or "an" does not exclude the possibility that more than one element is present, unless the context clearly requires that there be one and only one element. The indefinite article "a" or "an" thus usually means "at least one." Likewise, the terms "have," "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. For example, the expressions "A has B," "A comprises B" and "A includes B" may refer both to a situation in which, besides B, no other element is present in A *(i.e.,* a situation in which A solely and exclusively consists of B) or to a situation in which, besides B, one or more further elements are present in A, such as element C, elements C and D, or even further elements.

As used herein, the term Person with Diabetes (PwD) refers to a patient who is diagnosed with or is at-risk for being diagnosed with one or more forms of diabetes including of pre-diabetes, type 1 diabetes, type 2 diabetes, gestational diabetes, as well as one or more comorbidities that are associated with diabetes. Some embodiments described herein make more specific reference to insulin-dependent PwDs. Insulin-dependent PwDs administer insulin or similar medications that control levels of glucose, and some insulin-dependent PwDs administer multiple daily doses of insulin to manage increases in glucose levels that occur due to the consumption of meals. An individual dose of insulin is referred to as a bolus, and a diabetes management application allows the collection and review of diabetes logbook information and may include a bolus calculator to assist the PwD in determining the size of each bolus to maintain a healthy in-range glucose level. While the precise glucose values that are considered in-range for a particular PwD may vary, general guidelines that are known to the art recommend maintaining a glucose level within a range that does not exceed 140 or 180 mg/dL for a postprandial state or 100 mg/dL when fasting to avoid hyperglycemia and that does not fall below 70 mg/dL to avoid hypoglycemia.

As used herein, the term "physiological parameter" refers to any quantifiable aspect of a PwD's physiology that is measured as part of providing diabetes management services to a PwD. A non-limiting list of physiological parameters that are of interest to the treatment of diabetes and diabetes comorbidities includes glucose, glycosylated hemoglobin (HbA1c), ketones, estimated glomerular filtration rate (eGFR), and blood pressure (BP).

As used herein, the term "meal event" refers to an event in which a PwD consumes a meal and administers a bolus of insulin or a similar medication that regulates the postprandial glucose levels with the goal to keep the PwD in-range. A diabetes management application records a meal event as one in which the consumption of the meal itself is recorded, and in which a record of the medication bolus that the PwD administered with the meal is recorded. The diabetes management application may receive the data for the identified meal event contemporaneously to or later than the consumption of the meal and the mealtime medication bolus. As used herein, the term "unrecorded meal event" refers to an event in which the PwD consumes a meal and administers a bolus of medication, but does not enter a record of at least one of the meal or medication bolus into a diabetes management application. As used herein, the term "missed meal event" refers to an event in which the PwD consumes a meal but either administers no bolus of medication or administers an insufficient amount of medication, which leads to a postprandial hyperglycemic condition in the PwD. In some instances, the diabetes management application does not receive a record of the missed meal, while in other instances the diabetes management application receives a record that the missed meal was consumed, but the PwD fails to administer a proper medication bolus to control the postprandial glycemic response to the meal. As used herein, the term "recovery meal event" refers to a meal or medication that the PwD administers to recover from a hypoglycemic episode. A recovery meal can include the consumption of food, particularly food that is high in glucose, the administration of a medication such as glucagon that raises glucose levels, or both. The recovery meal event returns the PwD's glucose level to an acceptable postprandial in-range level.

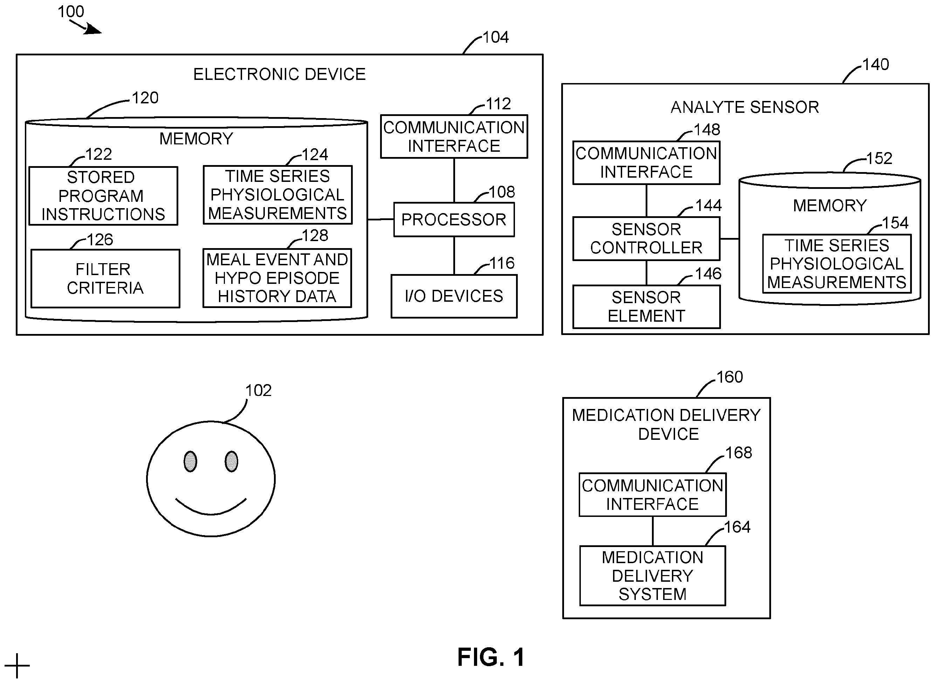

Fig. 1 depicts a diabetes management system 100 that includes an electronic device 104, an analyte sensor 140, and an optional medication delivery device 160. In FIG. 1, a user 102 is an insulin-dependent PwD who uses the diabetes management system 100 to manage his or her diabetes, including using the diabetes management system 100 to receive bolus recommendations for insulin or another diabetic medication.

In the system 100, the electronic device 104 includes an electronic device processor 108 that is operatively connected to a communication interface 112, input/output (I/O) devices 116, and a memory 120. While the specific attributes and operations of certain components in the electronic device 104 that are relevant to detecting and recording meal events and generating medication bolus recommendations are described in further detail herein, in at least some embodiments the electronic device 104 is embodied as a commercially available smartphone, tablet computer, personal computer, a wearable device such as a smartwatch or fitness tracker, or another suitable electronic device that the user 102 operates.

In the electronic device 104, the processor 108 is a digital logic device formed from one or more integrated circuits that typically includes one or more central processing unit (CPU) cores and a graphical processing unit (GPU) to execute stored program instructions that implement an operating system and application software and to provide a graphical user interface via one or more display devices, which are incorporated in the I/O devices 116 in FIG. 1. Amongst other software programs, a diabetes management application that is configured to receive data from the analyte sensor 140, provide diabetes management advice and bolus recommendations to the user 102 via the I/O devices 116, and optionally to send bolus commands to the medication delivery device 160 is one example of a software program that the processor 108 executes during operation. The diabetes management application is provided to the electronic device 104 via an online application store, an installation medium such as an optical disc or flash memory device, or through any other installation process known to the art. The processor 108 further incorporates peripheral device and memory controllers that provide operative connections to the communication interface 112, I/O devices 116, and the memory 120. Additional elements of the processor 108 optionally include digital signal processors (DSPs), image processing units, neural network accelerators, analog-to-digital converters, digital-to-analog converters, and any other suitable digital and analog electronic components. FIG. 1 depicts the processor 108, communication interface 112, I/O devices 116, and memory 120 as separate components for explanatory purposes, but in some embodiments the processor 108 is implemented with a System on a Chip (SoC) configuration that incorporates some or all of the components of the communication interface 112, I/O devices 116, and the memory 120.

In the electronic device 104, the communication interface 112 is, for example, a wireless or wired peripheral interface that provides a direct communication channel between the electronic device 104, the analyte sensor 140, and the medication delivery device 160. Non-limiting examples of wireless communication interface devices include a Bluetooth transceiver that implements one or more of the Bluetooth family of protocols including Bluetooth low-energy (BLE) and a near field communication (NFC) adapter. A non-limiting example of a wired communication interface device includes a universal serial bus (USB) adapter. Additionally, alternative communication interface embodiments including wired or wireless network interface adapters that provide a compatible communication interface between the electronic device 104, the analyte sensor 140, and the medication delivery device 160 may be used as well.

In the electronic device 104, the input/output [I/O] devices 116 include any hardware interfaces that enable the user 102 to receive information from and provide input to the diabetes management application and other software programs that the processor 108 executes during operation. Examples of input devices include input button, scroll wheel, keyboard, mouse, touchscreen, audio microphone input, camera devices, and the like. Examples of output devices include display screens, audio speakers, indicator lights, haptic feedback devices, and the like. During operation, the I/O devices 116 receive input from the user 102 to control and provide manual feedback to the diabetes management application, and the I/O devices provide outputs to the user 102 to provide bolus calculations and other diabetes management information.

In the electronic device 104, the memory 120 includes one or more volatile data storage devices such as random access memory (RAM) and non-volatile data storage devices such as solid state memory, magnetic, or optical media that store programmed instructions and data to control the operation of the electronic device 104. In particular, FIG. 1 depicts the memory 120 stored program instructions 122 that implement a diabetes management application to perform the processes described herein. The stored program instructions 122 also include an operating system and other associated commercially available software that control the operation of the electronic device 104. The memory 120 also stores time series data of physiological measurements 124, filter criteria 126, and a history of meal event and hypoglycemic episode data 128 that the diabetes management application uses to provide bolus calculations and other diabetes management recommendations to the user 102.

While the electronic device 104 is depicted as a self-contained electronic device that is capable of performing all of the functions and operations described herein, those of skill in the art will recognize that a more complex networked computing system may be employed to perform some of the functions and operations of the electronic device 104. For example, a network server computing system that is coupled to the electronic device 104 through a data network such as the Internet or another suitable network may receive the time series physiological measurements data 124, meal event and hypo event history data 128, and other data from the electronic device 104 and execute stored program instructions for the diabetes management program remotely to identify unrecorded meal events, missed meal events, recovery meal events, hypoglycemic episodes, and to generate medication bolus recommendations and other diabetes management program advice. The mobile electronic device 104 receives the results of these calculations and displays the results to the user 102 or performs other functions based on the results. Furthermore, in some configurations a remote data storage server stores backup copies or larger historical archives of the time series physiological measurements data 124, meal event and hypo event history data 128, and other data from the electronic device 104. As such, any reference to the operations of the processor 108, memory 120, or other elements of the electronic device 104 herein should be understood to be equally applicable to multiple computing systems that are communicatively coupled and configured to perform the functions and operations described herein.

In the system 100, the analyte sensor 140 is a device that generates physiological measurements of at least one chemical analyte in the body of the user 102. In most instances, the chemical analyte being measured is a level of glucose, and more particularly blood glucose, which the analyte sensor 140 measures directly or indirectly. In other configurations, the analyte sensor 140 detects other analytes such as ketones. In one embodiment, the analyte sensor 140 is a commercially available continuous glucose monitor (CGM) device that is affixed to the body of the user 102. The CGM analyte sensor 140 measures glucose values indirectly via interstitial fluid in the body of the user 102. A CGM analyte sensor 140 generates glucose measurements at regular intervals such as every minute, every five minutes, or at another periodic rate that enables the generation of a time series of physiological measurements, such as a time series of glucose level measurements in the user 102.

In the system 100, the analyte sensor 140 includes a sensor controller 144, a sensor element 146, a communication interface 148, and a memory 152. The sensor controller 144 is a digital, analog, or a combined digital/analog control device that is operatively connected to the sensor element 146, communication interface 148, and the memory 152. In most embodiments, the sensor controller 144 executes firmware instructions stored in the memory 152 (not shown) during operation. The sensor element 146 includes any electrochemical or other sensing hardware that perform detection of the analyte in the body of the user 102 to generate each physiological measurement. The sensor controller 144 operates the sensor element 146 to generate measurements of the physiological parameter, such as glucose, and stores one or more physiological parameter measurements in the memory 152 as a set of time series physiological measurements 154. The communication interface 148 in the analyte sensor 140 is another communication interface device that is compatible with the corresponding communication interface 112 in the electronic device 104. During operation, the sensor controller 144 operates the communication interface 148 to transmit the stored time series physiological measurements 154 to the electronic device 104. The electronic device processor 108 stores the data as the time series physiological measurement data 124 in the memory 120. In one operating mode, the sensor controller 144 transmits each physiological measurement datum to the electronic device 104 with minimal delay after generation of each measurement, while in other embodiments the memory 152 stores a set of multiple measurements in a time series and transmits batches of multiple physiological measurements to the electronic device 104 either periodically or in response to an on-demand request from the electronic device 104. Each physiological measurement datum in the physiological measurements 154 includes data corresponding to the measurement of the analyte, such as a quantitative value for the measured glucose level in the user 102, a timestamp indicating when the measurement was generated, and any other metadata necessary to track the measured analyte physiological parameters in the user 102 over time.

In the system 100, the medication delivery device 160 is a pump, smart pen, or other medication delivery device that is configured to receive a command from the electronic device 104 to deliver a recommended bolus of a medication, such as insulin, to the user 102. The medication delivery device 160 includes a medication delivery system 164 that further includes a reservoir for the medication, electromechanical devices that control the rate and amount of medication delivered to the user 102, and an electronic control device that operates the components of the medication delivery device and is operatively connected to a communication interface 168. The communication interface 168 in the medication delivery device 160 is another communication interface device that is compatible with the corresponding communication interface 112 in the electronic device 104. The medication delivery device 160 is an optional component of the system 100 because, in some configurations, the electronic device 104 provides a medication bolus recommendation to the user 102, and the user 102 subsequently doses the medication to the recommended bolus manually.

FIG. 1 describes the system 100 for illustrative purposes, but those of skill in the art will recognize that numerous alternative hardware and software configurations are suitable to implement the functions described herein. For example, some or all of the functionality of the electronic device 104 from FIG. 1 may be alternatively implemented in the analyte sensor 140 or the medication delivery device 160. Additionally, the functions attributed to the processor 108 in the electronic device 104 need not be fully implemented in a self-contained device but may instead be performed, at least in part, by one or more processors in network-connected servers, and any description of a single processor herein is understood to encompass multiple processor devices that are orchestrated to implement the functions described herein. Consequently, while the description herein makes specific references to the various components and processors in the system 100, those of skill in the art will recognize that these descriptions are not limiting in scope and are equally applicable to various hardware and software configurations.

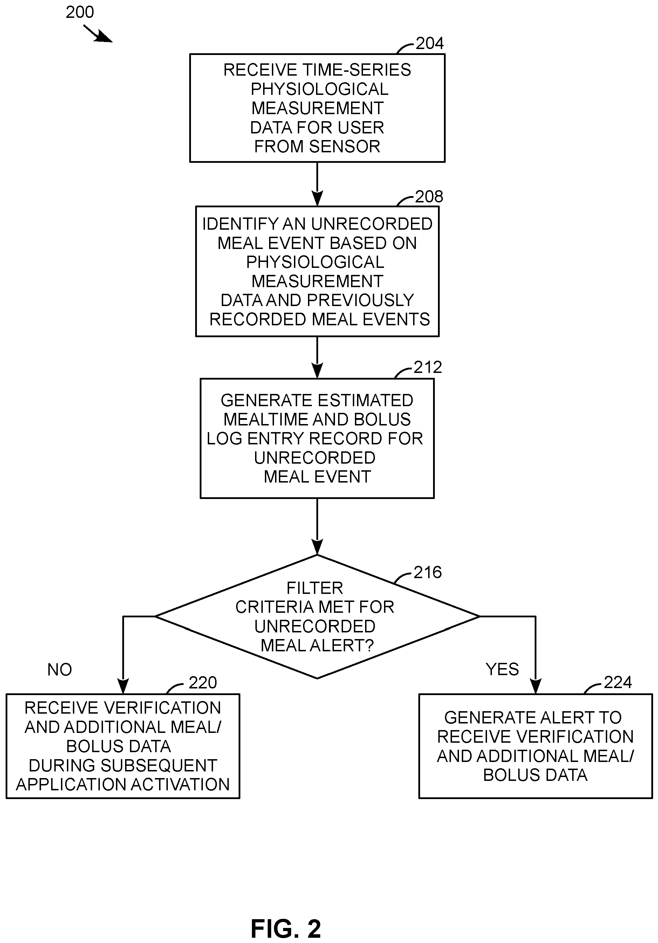

FIG. 2 is a block diagram of a process 200 for identification of unrecorded meal events and the automated generation of logs to record the unrecorded meal events in a diabetes management system. In the description below a reference to a function or operation of process 200 refers to the operation of one or more processors to execute stored program instructions to perform the function or operation in conjunction with other components in a diabetes management system. Process 200 is described in conjunction with the system 100 of FIG. 1 for illustrative purposes. In particular, while the stored program instructions 122 implement a diabetes management application that enables the user 102 to record meals and medication boluses in a conventional manner, the system 100 implements the additional functionality of process 200 to enable the automatic detection and logging of unrecorded meal events.

Process 200 begins as the electronic device 104 receives time series data of a measured physiological parameter from the analyte sensor 140 (block 204). In the system 100, the analyte sensor 140 generates the physiological measurements as a time series of glucose measurements or measurements of another analyte in the body of the user 102. The sensor controller 144 operates the communication interface 148 to transmit recorded physiological measurement values and corresponding timestamps to the corresponding communication interface 112 of the electronic device 104. As described above, in some configurations the analyte sensor 140 transmits the physiological measurement data with minimal delay while in other configurations the analyte sensor memory 152 stores data for a time series of multiple physiological measurements 154 that span a longer time period (e.g. multiple minutes or hours). In the electronic device 104, the processor 108 stores the glucose measurement data as the time series physiological measurement data 124 in the memory 120. In some embodiments, the electronic device 104 stores a larger set of the physiological data spanning days, weeks, or months of recorded physiological measurement data while the analyte sensor 140 stores a comparatively small amount of the physiological measurement data that are deleted after transmission of the data to the electronic device 104.

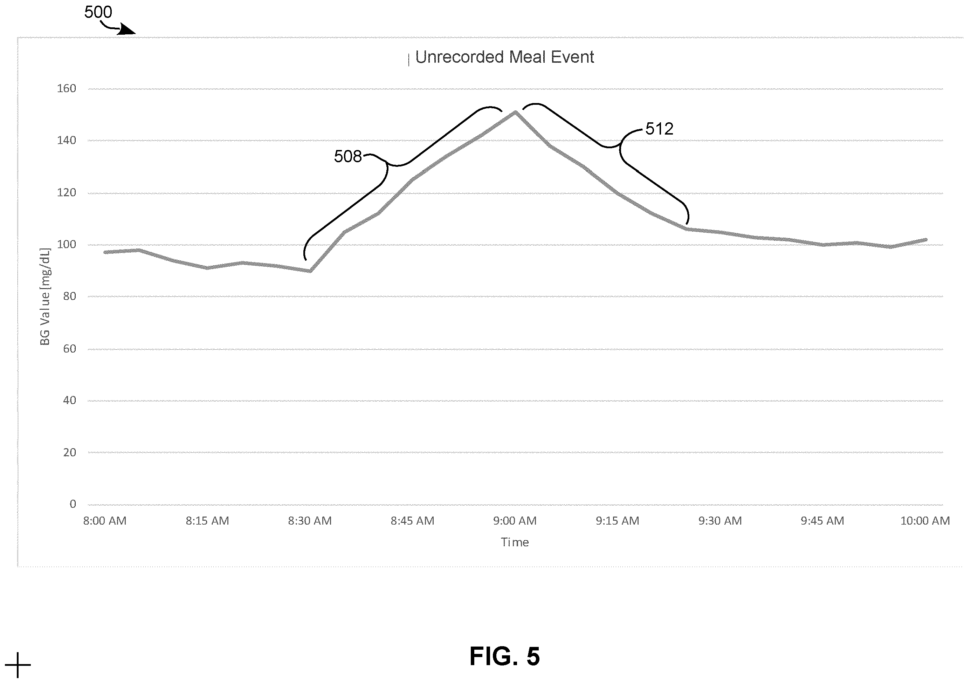

Process 200 continues as the electronic device 104 identifies data corresponding to at least one unrecorded meal event based on the glucose data or other physiological measurement data and on previously recorded meal events (block 208). In one configuration, the processor 108 in the mobile electronic device 104 automatically identifies a meal event based on an observed upward trend in the physiological measurement data for the user 102, and in particular to an increase in observed glucose levels. FIG. 5 depicts a graph 500 that plots a time series of glucose values corresponding to a meal event, which for purposes of process 200 is considered an unrecorded meal event when the user 102 does not provide an input to the system 100 that logs the meal and administered medication bolus. In one embodiment, the processor 108 identifies the unrecorded meal event based on a linear slope approximation or other derivative of the upward trend in glucose levels that occur within a predetermined time period after the meal as depicted in region 508, and generates a timestamp of the mealtime (e.g. at 8:30 AM) corresponding to the upward glucose trend in the region 508. Alternative meal detection techniques may be adapted to use with the diabetes management software in the system 100 as well. An exemplary algorithm is described by Turksoy, et al. in "Meal-Detection in Patients with Type 1 Diabetes: A New Module for The Multivariable Adaptive Artificial Pancreas Control System", IEEE J Biomed Health Inform (Jan. 2016). Still other techniques for meal detection include the use of artificial neural networks or other machine learning classifiers that are trained to identify physiological parameter patterns in time series data that correspond to meals from predetermined training data. In one embodiment, the timestamp of the estimated mealtime is a single time value, while in another embodiment the timestamp includes a time range that provides an estimate of the elapsed period of the meal. The processor 108 also identifies the downward trend in measured postprandial glucose values 512 that depicts a return to an in-range glucose level for the user 102, which indicates that the user 102 administered a medication bolus with the meal. The downward trend in the physiological parameter data, such as glucose levels in FIG. 5, indicate the physiological reaction of the user 102 to the administration of a medication bolus during the unrecorded meal event. The processor 108 compares the estimated timestamp of the detected meal event with previously recorded meal event timestamps in the meal event data 128 in the memory 120 to determine that the detected meal event is an unrecorded meal event when no previously recorded meal event has the same or similar timestamp.

Process 200 continues as the processor 108 generates an estimated mealtime and medication bolus log entry record for the identified unrecorded meal (block 212). In one configuration, the processor 108 uses the estimated timestamp of the unrecorded meal event to generate a record of the meal, and the system 100 receives further data regarding the size and contents of the meal and the size of the medication bolus from the user 102 at a later time. In another configuration, the processor 108 optionally estimates the nutritional contents of the meal in terms of total calories, carbohydrates/fats/proteins, or any other suitable nutrition metric. In one embodiment, the processor 108 generates the estimate based upon the magnitude of the increase in glucose levels that are present in the physiological measurement data. In another embodiment, the processor 108 uses a previously trained machine learning (ML) predictive model that generates the estimates based on the physiological measurement data in the time range of the unrecorded meal. In one configuration, a medication delivery device 160 such as an insulin pump or insulin smart pen records the sizes of an administered medication bolus and transmits the bolus size to the electronic device 104. The processor 108 stores the bolus data with the meal event data 128 and optionally may use the time of the bolus delivery to assist in determined the time of an unrecorded meal event. In other configurations where the precise medication bolus data are not available, the processor optionally generates an estimate of the administered bolus for the unrecorded meal event using a pre-existing bolus calculation algorithm. The bolus calculation algorithm uses the physiological measurement data of the elevation in glucose in the time range of the unrecorded meal relative to a target in-range glucose level, a predetermined insulin sensitivity factor for the user 102, and the estimated nutritional information for the unrecorded meal to determine an estimate of the administered medication bolus.

Process 200 continues as the processor 108 applies one or more filter criteria to the unrecorded meal event to identify if the unrecorded meal event satisfies one or more filter criteria to enable the generation of an alert to prompt the user 102 to enter a verification or correction to the estimated record for the unrecorded meal event (block 216). In the electronic device 104, the memory 120 stores filter criteria data 126 that include predefined or dynamically configured rules to control when the diabetes management application generates a prompt for the user 102 to provide information about an unrecorded meal.. In the embodiment of FIG. 1, the filter criteria data 126 include a time of day filter criterion that corresponds to the sleep schedule for the user 102. The sleep schedule filter criterion may be set by default for the diabetes management application or the user 102 may enter a sleep schedule criterion to update the criterion value dynamically. Examples of other filter criteria include requiring a minimum elapsed time from the most recent prior alert prior generating a new alert to reduce user fatigue during operation of the system 100 and filter criteria that suppress the alert regarding the unrecorded meal when another meal event or medication bolus is predicted to occur within a threshold time period when the user 102 is already expected to interact with the system 100.

If the processor 108 in the electronic device 104 identifies that the user 102 is most likely asleep based on the current time or that one or more other filter criteria are not met (block 216), then the processor 108 waits for the next activation of the diabetes management application in the system 100 to receive verification or additional information about the generated unrecorded meal event (block 220). In some configurations, the diabetes management application presents a confirmation output to the user 102 when the user 102 next activates the diabetes management application to verify that the meal event occurred at the identified time, and optionally to enable the user 102 to provide additional information about the meal event that the processor 108 stores with the meal event data 128 or correct the automatically generated estimated information about the meal event. As described above, the additional information about the meal includes elements such as an estimate of the meal size, total number of calories, the proportions of carbohydrates/proteins/fats in the meal, or any other suitable information about the meal. If the user 102 did not consume a meal at detected time of the unrecorded meal, then the processor 108 either deletes the unrecorded meal event record or prompts the user 102 to input a corrected time if an unrecorded meal event occurred at a different time from the automatically identified time of the unrecorded meal event.

If the processor 108 in the electronic device 104 identifies that the filter criteria are met (block 216), then the processor 108 generates an alert to receive verification and additional information about the generated unrecorded meal event (block 224). In this configuration, the processor 108 generates a visual, audio, audiovisual, or haptic alert to the user 102 via the I/O devices 116 in the electronic device 104. The electronic 104 receives the verification of the unrecorded meal event and additional data from the user 102 in a similar manner to that described above with reference to the processing of block 220.

The electronic device 104 generates an output that informs the user 102 of the identified meal event and the user 102 confirms the consumption of the meal at the timestamp for the identified meal, optional details about the meal, and the dosage of the administered medication bolus. If the user 102 did not consume a meal within the general time frame of the detect meal or did not administer the estimated medication bolus, then the diabetes management application receives a negation of the meal event from the user and either deletes the meal event or receives input from the user 102 to modify information pertaining to the meal event. In another configuration, the electronic device 104 receives meal event data as an input from the user 102 via the I/O devices 116 that indicates the time of consumption of the meal and optionally includes information about the meal such as an estimate of the meal size, total number of calories, the proportions of carbohydrates/proteins/fats in the meal, or any other suitable information about the meal.

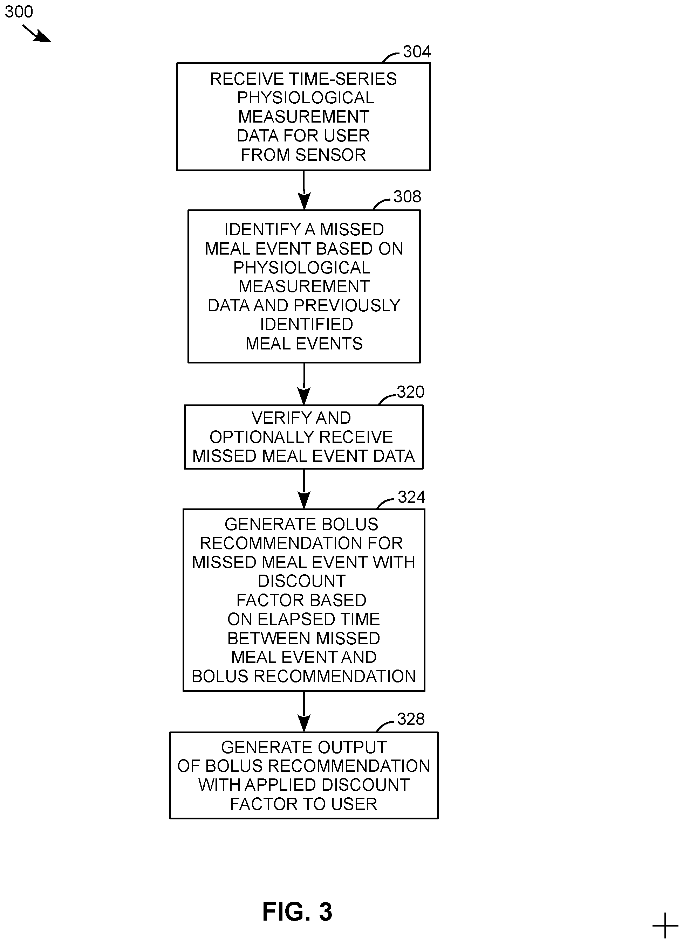

FIG. 3 is a block diagram of a process 300 for identification of missed meal events and the generation of medication bolus recommendations in a diabetes management system. In the description below a reference to a function or operation of process 300 refers to the operation of one or more processors to execute stored program instructions to perform the function or operation in conjunction with other components in a diabetes management system. Process 300 is described in conjunction with the system 100 of FIG. 1 for illustrative purposes. In the system 100, the processor 108 in the electronic device 104 may implement process 300 concurrently with process 200 described above, or be configured to perform either process separately.

Process 300 begins as the electronic device 104 receives time series data of a measured physiological parameter from the analyte sensor 140 (block 304). During process 300 the electronic device 104 receives the physiological parameter data, such as glucose data, from the analyte sensor 140 in substantially the same manner as described above with reference to the processing of block 204 in process 200. In some embodiments, the processor 108 performs processes 200 and 300 simultaneously using a common set of physiological parameter data received from the analyte sensor 140.

Process 300 continues as the processor 108 identifies a missed meal event based on the physiological parameter data and records of previously recorded meals (block 308). While the user 102 may record most meal events, in some instances the user 102 consumes a meal without administering a proper bolus of insulin or other medication with the meal to control postprandial glucose levels, which results in a hyperglycemic condition. In the electronic device 104, the processor 108 identifies a missed meal event based on both the received physiological measurement data, such as the time series glucose data, and on the records of previously identified meal events to avoid an inaccurate association of the missed meal event with a previously identified meal event. In some instances, the user 102 activates the diabetes management application in the electronic device 104 only on an intermittent basis, and the electronic device 104 receives a backlog of the physiological parameter data from the analyte sensor 140 to enable the processor 108 to identify a missed meal event in the time series physiological parameter measurements, such as a time series of glucose measurements.

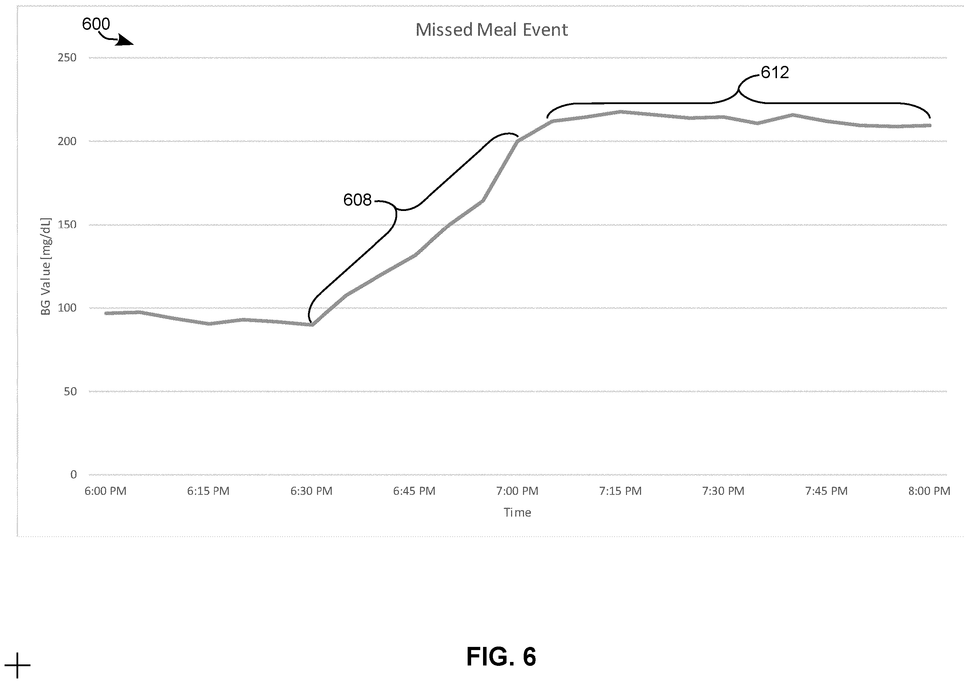

FIG. 6 depicts a graph 600 of that plots a time series of glucose values corresponding to a missed meal event. The processor 108 identifies the meal event based on a linear slope approximation or other derivative of the upward trend in glucose levels that occur within a predetermined time period after the meal as depicted in region 608 or using another meal detection algorithm that is otherwise known to the art in a similar manner to the unrecorded meal detection process that is described above with reference to the processing of block 208 in process 200. The processor 108 generates a timestamp of the mealtime at a time (e.g. 6:30 PM) corresponding to an upward glucose trend region 608 in a similar manner to that described above with reference to the graph 500 of FIG. 5. The processor 108 identifies that the meal event is a missed meal event, instead of a recorded meal event, unrecorded meal event, or a recovery meal event, based both on the timestamp for the estimated time of the meal, which does not correspond to the timestamp of an identified meal event or recorded bolus, and based on the postprandial glucose data that remain elevated above a target range threshold subsequent to the missed meal event timestamp without showing signs of returning to the in-range levels that would occur if the user 102 administers a proper bolus of medication. In FIG. 6, the range 612 indicates a prolonged period of elevated glucose levels in the physiological measurement data that exceed a predetermined maximum threshold (e.g. 120 mg/dL) without indicating a downward progression into an in-range glucose level, and the processor 108 identifies missed meal event based in part on this postprandial response.

Referring again to FIG. 3, after identification of a missed meal event, the processor 108 generates an alert to inform the user 102 of the missed meal and optionally receives a verification of the missed meal and additional data corresponding to the time and nutritional content missed meal event (block 320). In the mobile electronic device 104, the processor 108 records the time of the meal event in association with the meal event data 128. The diabetes management application also presents a confirmation output to the user 102 to confirm that the meal event occurred at the identified time, and optionally to enable the user 102 to provide additional information about the meal event that the processor 108 stores with the meal event data 128. In some configurations, the user 102 optionally updates the timestamp of the missed meal event if the automatically generated timestamp is inaccurate but the user did consume a meal in the general timeframe indicated by the glucose data. If, however, the user 102 did not consume a meal corresponding the missed meal event, then the user 102 enters a negation in response to the confirmation output and the diabetes management application cancels any mealtime bolus calculations and does not record the missed meal event.

During process 300, if the user 102 confirms the missed meal event, then the processor 108 executes the diabetes management application to generate a bolus recommendation for the missed meal event that applies a time discounting factor based upon a length of time elapsed from the missed meal event to a current time (block 324). Using the system 100 of FIG. 1 as an example, the processor 108 utilizes a prior art bolus calculation algorithm to generate a recommended bolus size for the user 102 as if the user 102 had entered the meal time information to calculate a medication bolus at the time of the meal, such as calculating the meal time bolus during an identified meal event. As such, the diabetes management application may utilize an existing bolus calculation algorithm to generate the mealtime bolus recommendation. Because a missed meal event occurs after the consumption of a meal, during process 300, the processor 108 identifies a length of time elapsed from the missed meal event time to the current time and applies a time-varying discount factor to the recommended bolus size, which typically adjusts the size of the recommended mealtime bolus downward to account for the elapsed time since the missed meal event occurred. The discount factor described herein is relative to a difference between a mealtime bolus, which is typically a larger medication bolus that accounts for an intake of carbohydrates, and a correction bolus, which is a medication bolus that is calculated to decrease a hyperglycemic glucose level to a predetermined in-range glucose level for a PwD without taking into account a particular intake of carbohydrates and is typically a smaller medication bolus than the mealtime bolus. In one example, the processor 108 uses the following time discount factor table in the diabetes management application:

**Table 1: Example time varying discount factors**

| Elapsed Time After Missed Meal Event | Discount Factor Mealtime Bolus → Correction bolus |
|---|---|
| 0 min (theoretical) | No discount - 100% (full mealtime bolus) |
| 15 min | 75% |
| 30 min | 50% |
| 45 min | 25% |
| 60 min + | Fully discounted 0% (correction bolus only) |

Table 1 depicts a linear discount factor that is applied to the size of the recommended meal time bolus from the bolus calculation algorithm based on the elapsed time from a timestamp of missed meal event, with a short delay (0 to 15 minutes) resulting in a recommendation for the full meal time bolus (100%), and gradually longer delays resulting in a percentage scaling of the recommended mealtime bolus size factor down to 0% (only calculate a standard correction bolus) after 60 minutes. For example, if the undiscounted calculated mealtime bolus is 8 units of insulin and a calculated correction bolus is 4 units of insulin, then a 50% discount factor yields a recommended 6 units of insulin bolus when the bolus is calculated 30 minutes after the meal is consumed. Of course, the discount factors of Table 1 are merely illustrative examples, and alternative configurations that use discount factors based on discrete or continuous time linear decay functions, piecewise decay functions, exponential decay functions, logarithmic decay functions, or any other suitable decay function. Additionally, alternative configurations may use longer or shorter discount time horizons that the 60 minute time horizon that is depicted in Table 1. In situations where the elapsed time from the timestamp of the missed meal event exceeds the maximum time threshold for a mealtime bolus calculation, the diabetes management application optionally generates a correction bolus calculation if the user 102 remains in a hyperglycemic state. The correction bolus calculation typically does not use specific data about a consumed meal in the bolus calculation, but instead calculates a bolus based on the currently measured glucose levels, predetermined insulin sensitivity of the user 102, the desired target glucose level to return the user 102 to in-range glucose levels, and the prior history of meals and boluses to provide a safe and effective correction bolus.

Process 300 continues as the processor 108 generates an output message indicating the recommended bolus to the user 102 and optionally to the medication delivery device 160 to deliver a recommended bolus of insulin or another medication to the user 102 (block 328). In the electronic device 104, the processor 108 operates one or more of the I/O devices 116 to produce an output message that indicates the recommended bolus of the medication to the user 102. In one configuration, a display device in the electronic device 104 generates a visual display of the recommended bolus, while in another configuration the processor 108 uses speech synthesis hardware and software to generate an audible output of the recommended bolus using an audio speaker, and the electronic device 104 optionally generates the output using multiple output devices. In configurations of the system 100 that include the medication delivery device 160, the electronic device 104 optionally transmits a command message to the medication delivery device 160 to operate the medication delivery device 160 to administer the recommended bolus of insulin or other medication to the user 102. For example, the processor 108 uses the communication interface 112 to transmit a command message to the corresponding communication interface 168 in the medication delivery device 160. The user 102 injects the medication in the set bolus dose based on the command message using a smart pen medication device, or if the medication delivery device 160 is a medication pump, then the pump 160 delivers the bolus in response to the command message from the electronic device 104.

Processes 200 and 300 are typically, although not exclusively, conducted in response to meals that the user 102 consumes as part of his or her daily schedule. In other situations, a PwD consumes a recovery meal to return his or her glucose levels to a healthy in-range level in response to a hypoglycemic episode. In most situations, the PwD does not need to administer a bolus of insulin or other medication due to a recovery meal, but the diabetes management application stores a record of the hypoglycemic episode and the recovery meal to assist the PwD and healthcare providers in long-term glucose management to avoid future hypoglycemic episodes. As such, ensuring that the diabetes management application receives accurate information about recovery meals provides medical benefits to the user 102.

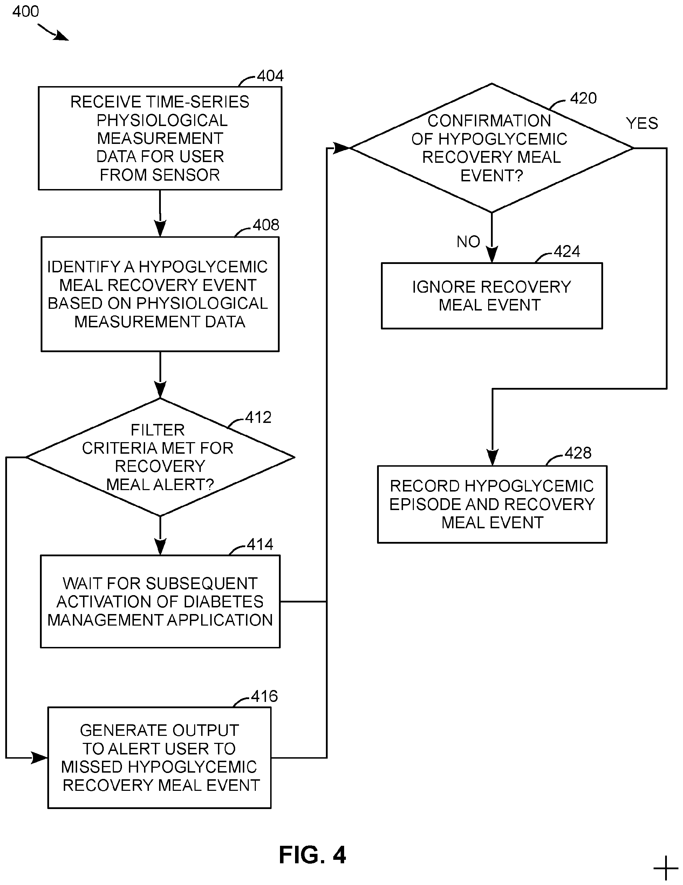

FIG. 4 is a block diagram of a process 400 for detecting and recording recovery meals that a PwD consumes to recover from hypoglycemic episodes in a diabetes management system. In the description below a reference to a function or operation of process 300 refers to the operation of one or more processors to execute stored program instructions to perform the function or operation in conjunction with other components in a diabetes management system. Process 400 is described in conjunction with the system 100 of FIG. 1 for illustrative purposes. In the system 100, the processor 108 in the electronic device 104 may implement process 400 concurrently with either or both of processes 200 and 300 described above, or be configured to perform any of these processes separately.

Process 400 begins as the electronic device 104 receives time series data of a measured physiological parameter from the analyte sensor 140 (block 404). During process 400, the electronic device 104 receives the physiological parameter data, such as glucose data, from the analyte sensor 140 in substantially the same manner as described above with reference to the processing of block 204 in process 200. In some embodiments, the processor 108 performs one or more of the processes 200, 300, and 400 simultaneously using a common set of physiological parameter data received from the analyte sensor 140.

Process 400 continues as the processor 108 in the electronic device 104 identifies a hypoglycemic recovery meal event based on the physiological measurement data and the at least one meal event (block 408). While a hypoglycemic episode may occur at any time of day, many hypoglycemic episodes occur overnight while the PwD is sleeping, and hypoglycemic episodes often cause disorientation. As such, in many instances the user 102 may consume a recovery meal but will not use the diabetes management application to record the recovery meal at the time of the hypoglycemic episode. In the electronic device 104, the processor 108 identifies a recovery meal event based on both the received physiological measurement data, such as the time series glucose data, and on the records of previously identified meal events to avoid an inaccurate association of the recovery meal event with a previously identified meal event. In some instances, the user 102 activates the diabetes management application in the electronic device 104 only on an intermittent basis, and the electronic device 104 receives a backlog of the physiological parameter data from the analyte sensor 140 to enable the processor 108 to identify a recovery meal event in the time series physiological parameter measurements, such as a time series of glucose measurements.

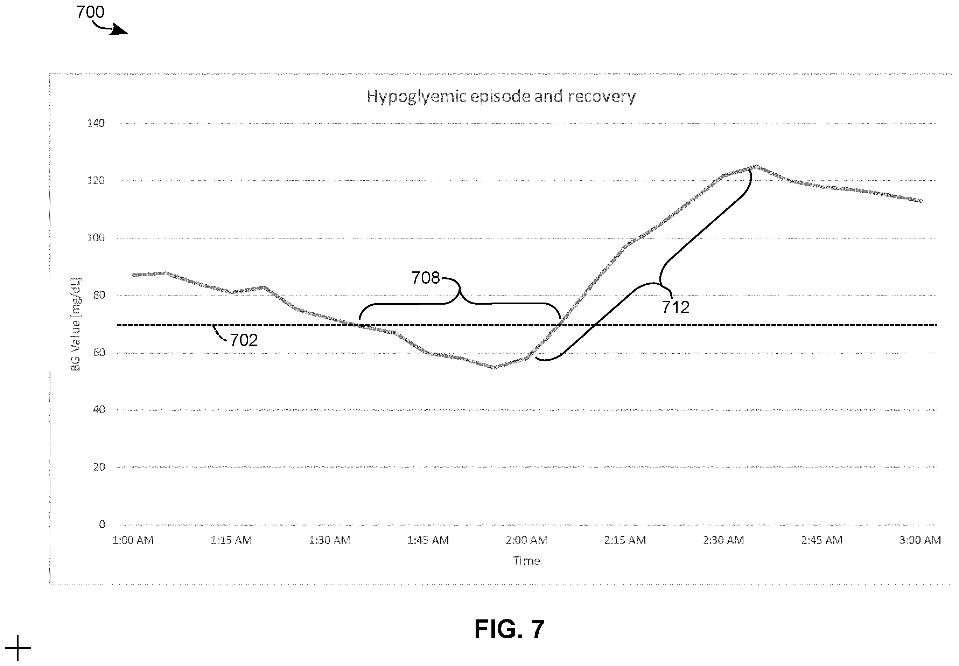

FIG. 7 depicts a graph 700 that plots a time series of glucose values corresponding to a recovery meal event. The processor 108 identifies the recovery meal event based on a minimum glucose level threshold 702 that indicates the user 102 has experienced a hypoglycemic event because the time series glucose data fall below the threshold. In the example of FIG. 7, the minimum glucose level threshold 702 is indicated as 70 mg/dL, but in the electronic device 104 the diabetes management application may be configured with a different threshold level. The processor 108 identifies the hypoglycemic episode based on the time series of glucose levels that fall below the minimum glucose threshold 702, which are indicated in the region 708 of the plot 700 in FIG. 7. The processor 108 uses one of the meal detection techniques that are described above in processes 200 and 300 to detect a meal in response to the increased glucose levels in the physiological parameter data as depicted in region 712 of the plot 700 to generate a timestamp for an estimate of the recovery mealtime. The processor 108 identifies that the meal event is a recovery meal event based both on the estimated time of the meal, which does not correspond to the time of a previously recorded meal event, and based on the measurement of the hypoglycemic episode in region 708 and the recovery from the hypoglycemic episode in region 712 of the time series glucose data. In particular, for a recovery meal event the processor 108 generates the timestamp for the estimated recovery meal as being within the hypoglycemic event time range 708 and corresponding to the upward trend in glucose levels as depicted in the region 712, and a return to an in-range glucose level. These features distinguish a recovery meal event from an unrecorded meal event or missed meal event.

Referring again to FIG. 4, during process 400 the processor 108 applies one or more filter criteria to the recovery meal event to identify if the recovery meal event satisfies one or more filter criteria to enable the generation of an immediate alert to prompt the user 102 to enter a verification or correction to the record for the identified recovery meal event (block 412). In the system 100, the processor 108 uses the filter criteria 126 to determine if the time or other aspects of the recovery meal event meet the requirements to generate an immediate alert or, if the criteria are not met, to wait for a subsequent activation of the diabetes management application to alert the user 102 of the recovery meal event. While the processor 108 applies the filter criteria 126 in processes 200 and 400 in a similar manner, in some embodiments the specific set of filter criteria rules that apply to unrecorded meal events may differ from the specific set of filter criteria rules that apply to recovery meal events.

If the identified recovery meal event does not meet applied filter criteria are met (block 412) then process 400 continues as the electronic device 104 does not generate an immediate output alert to the user 102 and instead waits for a subsequent activation of the diabetes management application to receive further confirmation of the hypoglycemic recovery meal event (block 414). If, however, the identified recovery meal event meets applied filter criteria are met (block 412) then process 400 continues as the electronic device 104 generates an output to alert the user 102 to the identification of the recovery meal event (block 416). In some configurations, the processor 108 generates the output using the I/O devices 116 when the user 102 next activates the diabetes management application after the hypoglycemic episode and recovery meal occur. In other configurations, the processor 108 generates the output using the I/O devices 116 at a preconfigured time, such as when the user 102 normally wakes up in the morning or at the next meal event for the user 102.

The output alert includes a confirmation prompt that enables the user 102 to confirm that a recovery meal event took place or to indicate that no recovery meal event took place. If the electronic device 104 receives a user input that does not confirm the recovery meal event (block 416), then the electronic device 104 ignores the recovery meal and the processor 108 makes no record of the recovery meal event in the meal event and hypoglycemic episode history data 128 (block 420). In at least some configurations, however, the processor 108 generates a record of the hypoglycemic event based on the physiological parameter data from the analyte sensor 140 even if the user 102 indicates that there was no recovery meal event, but this record does not include additional data pertaining to the recovery meal. If, however, the electronic device 104 receives a user input that confirms the recovery meal event (block 416) then the processor 108 stores a record of the hypoglycemic episode and the recovery meal event in the meal event and hypoglycemic episode history data 128 (block 424). As part of the record, the diabetes management application optionally prompts the user 102 to enter additional information about the recovery meal event, such as to input the type and quantity of food consumed, if the PwD administered a medication such a glucagon in addition to or instead of eating food, and to input any symptoms that were associated with the hypoglycemic episode. The electronic device 104 receives the input for the additional recovery meal event information via the I/O devices 116.

The system 100 and processes 200, 300, and 400 described above enable improvements to tracking the consumption of unrecorded meals, to the calculation of boluses for insulin or other medications when a PwD fails to administer the proper medication bolus with missed meals, and to detecting recovery meals in association with hypoglycemic episodes. These processes benefits to diabetes management for a PwD who uses the system 100 in making meal logging more efficient and providing dynamic updates to bolus recommendations in the event of a missed meal. These processes also improve the operation of the diabetes management software through greater accuracy in tracking meal events and hyperglycemic or hypoglycemic episodes, which improves the ability of the system 100 to provide accurate recommendations for diabetes management.

This disclosure is described in connection with what are considered to be the most practical and preferred embodiments. However, these embodiments are presented by way of illustration and the scope of this disclosure is not intended to be limited to the disclosed embodiments. Accordingly, one of skill in the art will realize that this disclosure encompasses all modifications and alternative arrangements within the spirit and scope of the disclosure and as set forth in the following numbered embodiments and claims.

### Numbered Embodiments:

1. A method for medication bolus calculation comprising:
   providing stored program instructions of a diabetes management application, the diabetes management application being configured to be stored in a memory of an electronic device and, upon execution by a processor in the electronic device, the diabetes management application being configured to:
   receive a plurality of physiological measurements from an analyte sensor, the plurality of physiological measurements being received as a time series of physiological measurements from the analyte sensor coupled to a user;
   identify a missed meal event corresponding to a meal consumed by the user, the diabetes management application being further configured to:
      generate a timestamp for the missed meal event based on a change in the plurality of physiological measurements corresponding to a time of the meal;
      identify an elevated level of the physiological measurements above a predetermined threshold occurring subsequent to the timestamp; and
      identify that the timestamp for the missed meal event does not correspond to a timestamp of any of one or more additional meal events stored in a memory operatively coupled to the processor;
   calculate a recommended bolus to administer in response to the missed meal event, the recommended bolus being calculated based, at least in part, upon an length of time elapsed from the timestamp of the missed meal event to a current time; and
   generate, with an output device coupled to the processor, an output message indicating the recommended bolus of the medication.
2. A computer program product comprising program instructions configured to operate a processor to:
   receive a plurality of physiological measurements from an analyte sensor, the plurality of physiological measurements being received as a time series of physiological measurements from the analyte sensor coupled to a user;
   identify a missed meal event corresponding to a meal consumed by the user, the processor being further configuring to:
      generate a timestamp for the missed meal event based on a change in the plurality of physiological measurements corresponding to a time of the meal;
      identify an elevated level of the physiological measurements above a predetermined threshold occurring subsequent to the timestamp; and
      identify that the timestamp for the missed meal event does not correspond to a timestamp of any of one or more additional meal events stored in a memory operatively coupled to the processor;
   calculate a recommended bolus to administer in response to the missed meal event, the recommended bolus being calculated based, at least in part, upon an length of time elapsed from the timestamp of the missed meal event to a current time; and
   generate with an output device coupled to the processor, an output message indicating the recommended bolus of the medication.
3. A method for unrecorded meal identification in a diabetes management system comprising:
   providing stored program instructions of a diabetes management application, the diabetes management application being configured to be stored in a memory of an electronic device and, upon execution by a processor in the electronic device, the diabetes management application being configured to:
   receive a plurality of physiological measurements from an analyte sensor, the plurality of physiological measurements being received as a time series of physiological measurements from the analyte sensor coupled to a user;
   identify a recovery meal event corresponding to a meal consumed by the user, the processor being further configuring to:
      generate a timestamp for the recovery meal event based on a change in the plurality of physiological measurements corresponding to a time of the meal;
      identify at least a portion of the plurality of physiological measurements occurring prior to the timestamp falling beneath a predetermined threshold; and
      identify that the timestamp for the recovery meal event does not correspond to a timestamp of any of one or more additional meal events stored in a memory operatively coupled to the processor;
   generate, with an output device, an output to alert the user to the recovery meal event; and
   store a record of the recovery meal event in a memory in response to receiving a confirmation via an input device coupled to the processor.
4. A computer program product comprising program instructions configured to operate a processor to:
   receive a plurality of physiological measurements from an analyte sensor, the plurality of physiological measurements being received as a time series of physiological measurements from the analyte sensor coupled to a user;
   identify a recovery meal event corresponding to a meal consumed by the user, the processor being further configuring to:
      generate a timestamp for the recovery meal event based on a change in the plurality of physiological measurements corresponding to a time of the meal;
      identify at least a portion of the plurality of physiological measurements occurring prior to the timestamp falling beneath a predetermined threshold; and
      identify that the timestamp for the recovery meal event does not correspond to a timestamp of any of one or more additional meal events stored in a memory operatively coupled to the processor;
   generate, with an output device, an output to alert the user to the recovery meal event; and
   store a record of the recovery meal event in a memory in response to receiving a confirmation via an input device coupled to the processor.
5. A method for recovery meal event identification in a diabetes management system comprising:
   providing stored program instructions of a diabetes management application, the diabetes management application being configured to be stored in a memory of an electronic device and, upon execution by a processor in the electronic device, the diabetes management application being configured to:
   receive a plurality of physiological measurements from an analyte sensor, the plurality of physiological measurements being received as a time series of physiological measurements from the analyte sensor coupled to a user;
   identify a recovery meal event corresponding to a meal consumed by the user, the processor being further configured to:
      generate a timestamp for the recovery meal event based on a change in the plurality of physiological measurements corresponding to a time of the meal;
      identify at least a portion of the plurality of physiological measurements occurring prior to the timestamp falling beneath a predetermined threshold; and
      identify that the timestamp for the recovery meal event does not correspond to a timestamp of any of one or more additional meal events stored in a memory operatively coupled to the processor;
   generate, with an output device, an output to alert the user to the recovery meal event; and
   store a record of the recovery meal event in a memory in response to receiving a confirmation via an input device coupled to the processor.
6. A computer program product comprising program instructions configured to operate a processor to:
   receive a plurality of physiological measurements from an analyte sensor, the plurality of physiological measurements being received as a time series of physiological measurements from the analyte sensor coupled to a user;
   identify a recovery meal event corresponding to a meal consumed by the user, the processor being further configured to:
      generate a timestamp for the recovery meal event based on a change in the plurality of physiological measurements corresponding to a time of the meal;
      identify at least a portion of the plurality of physiological measurements occurring prior to the timestamp falling beneath a predetermined threshold; and
      identify that the timestamp for the recovery meal event does not correspond to a timestamp of any of one or more additional meal events stored in a memory operatively coupled to the processor;
   generate, with an output device, an output to alert the user to the recovery meal event; and
   store a record of the recovery meal event in a memory in response to receiving a confirmation via an input device coupled to the processor.

## Claims

1. A method for medication bolus calculation comprising:
receiving, with a processor, a plurality of physiological measurements from an analyte sensor, the plurality of physiological measurements being received as a time series of physiological measurements from the analyte sensor coupled to a user;
identifying, with the processor, a missed meal event corresponding to a meal consumed by the user, the identifying further comprising:
generating a timestamp for the missed meal event based on a change in the plurality of physiological measurements corresponding to a time of the meal;
identifying an elevated level of the physiological measurements above a predetermined threshold occurring subsequent to the timestamp; and
identifying that the timestamp for the missed meal event does not correspond to a timestamp of any of one or more additional meal events stored in a memory operatively coupled to the processor;
calculating, with the processor, a recommended bolus to administer in response to the missed meal event, the recommended bolus being calculated based, at least in part, upon an length of time elapsed from the timestamp of the missed meal event to a current time; and
generating, with an output device coupled to the processor, an output message indicating the recommended bolus of the medication.

2. The method of claim 1 further comprising:
generating, with the output device, a prompt indicating a timestamp of the missed meal event time to the user; and
receiving, with the processor, an input in response to the prompt, the input further comprising one of:
a confirmation that a meal was consumed at the timestamp of the missed meal event time included in the prompt or a timestamp received from the user; or
a negation indicating that no meal has been consumed other than the at least one meal event.

3. The method of claim 2, wherein the processor cancels the calculation of the recommended bolus in response to receiving the negation in the input.

4. The method of any of claims 1 - 3, the calculating of the recommended bolus further comprising:
applying a time-varying discount factor to the recommended bolus size, the time-varying discount factor decreasing the recommended bolus size proportionately to the length of time elapsed from the missed meal event time to the current time.

5. The method of any of claims 1 - 4, wherein the output message further comprises a command transmitted to a medication delivery device to deliver the recommended bolus to the patient.

6. The method of any of claims 1 - 5, wherein the medication is insulin.

7. The method of any of claims 1 - 6, wherein the analyte sensor is a continuous glucose monitor.

8. A method for unrecorded meal identification in a diabetes management system comprising:
receiving, with a processor, a plurality of physiological measurements from an analyte sensor, the plurality of physiological measurements being received as a time series of physiological measurements from the analyte sensor coupled to a user;
identifying, with the processor, an unrecorded meal event corresponding to a meal consumed by the user, the identifying further comprising:
generating a timestamp for the unrecorded meal event based on a change in the plurality of physiological measurements corresponding to a time of the meal;
identifying a reduction in the plurality of physiological measurements occurring subsequent to the timestamp corresponding to a physiological reaction to administration of a medication bolus; and
identifying that the timestamp for the unrecorded meal event does not correspond to a timestamp of any of one or more additional meal events stored in a memory operatively coupled to the processor;
generating, with an output device coupled to the processor, an output to alert the user to the unrecorded meal event; and
storing, with the processor, a record of the unrecorded meal event in a memory in response to receiving a confirmation via an input device coupled to the processor.

9. The method of claim 8 further comprising:
applying, with the processor, a filter criterion to the identified unrecorded meal event; and
generating the output alert at a time of the identification of the unrecorded meal event only in response to the applied filter criterion being met.

10. The method of claim 9, wherein the processor identifies that a filter criterion is not met and cancels generating the output alert in response to the identifying of the unrecorded meal event occurring during a predetermined sleep period for the user.

11. The method of any of claims 8 - 10, wherein the plurality of physiological measurements from the analyte sensor are a plurality of glucose measurements from a continuous glucose monitor.

12. The method of any of claims 8 - 11, wherein the medication is insulin.

13. A method for recovery meal event detection in a diabetes management system comprising:
receiving, with a processor, a plurality of physiological measurements from an analyte sensor, the plurality of physiological measurements being received as a time series of physiological measurements from the analyte sensor coupled to a user;
identifying, with the processor, a recovery meal event corresponding to a meal consumed by the user, the identifying further comprising:
generating a timestamp for the recovery meal event based on a change in the plurality of physiological measurements corresponding to a time of the meal;
identifying at least a portion of the plurality of physiological measurements occurring prior to the timestamp falling beneath a predetermined threshold; and
identifying that the timestamp for the recovery meal event does not correspond to a timestamp of any of one or more additional meal events stored in a memory operatively coupled to the processor;
generating, with an output device coupled to the processor, an output to alert the user to the recovery meal event; and
storing, with the processor, a record of the recovery meal event in a memory in response to receiving a confirmation via an input device coupled to the processor.

14. The method of claim 13 further comprising:
receiving, with the processor, input data corresponding to at least one of a type of food consumed, quantity of food consumed, or type of medication administered during the recovery meal event in response to the output alert; and
storing, with the processor, the input data in association with the record of the recovery meal in the memory.

15. The method of either of claims 13 - 14, wherein the plurality of physiological measurements from the analyte sensor are a plurality of glucose measurements from a continuous glucose monitor.
